# EUROPÄISCHE PATENTSCHRIFT

(11) **EP 0 813 526 B1**
(45) Veröffentlichungstag und Bekanntmachung des Hinweises auf die Patenterteilung: **04.02.2004**
(21) Anmeldenummer: 96905803.1
(22) Anmeldetag: 27.02.1996
(51) Int. Cl.: C07D 231/12

(54) **VERFAHREN ZUR HERSTELLUNG VON N-SUBSTITUIERTEN PYRAZOLEN**
PROCESS FOR PREPARING N-SUBSTITUTED PYRAZOLES
PROCEDE DE PREPARATION DE PYRAZOLS SUBSTITUES EN N

(30) Priorität: 04.03.1995 DE 19507600; 15.03.1995 DE 19509361; 18.03.1995 DE 19509958
(43) Veröffentlichungstag der Anmeldung: 29.12.1997
(73) Patentinhaber: BASF AKTIENGESELLSCHAFT, 67056 Ludwigshafen (DE)
(72) Erfinder: MERKLE, Hans, Rupert, D-67061 Ludwigshafen (DE); FRETSCHNER, Erich, D-69239 Neckarsteinach (DE); SCHRÖDER, Jürgen, D-67071 Ludwigshafen (DE)
(74) Vertreter: Kinzebach, Werner, Dr.
(86) Internationale Anmeldenummer: PCT/EP1996/000790
(87) Internationale Veröffentlichungsnummer: WO 1996/027589

(56) Entgegenhaltungen:
- EP-A- 0 628 563

## Beschreibung

Die vorliegende Erfindung betrifft ein Verfahren zur Herstellung von N-substituierten Pyrazolen durch Umsetzung von Pyrazolen mit Alkoholen in flüssiger Phase in Gegenwart von Katalysatoren bei erhöhten Temperaturen.

Aus der DE-A-35 06 972 und US-A-3,910,949 ist ein Verfahren zur N-Alkylierung von 3,5-Diphenylpyrazolen bekannt, bei dem N-unsubstituierte 3,5-Diphenylpyrazole mit Dimethylsulfat/wäßriger Natronlauge in Gegenwart eines Phasentransferkatalysators umgesetzt werden.

DE-A-24 25 979 beschreibt die Alkylierung von 3,5-Diarylpyrazolen mit Alkylhalogeniden bzw. Dialkylsulfaten.

Nachteilig bei diesen Verfahren ist der Einsatz der sehr giftigen und relativ teuren Dialkylsulfate, da der größere Teil dieses Moleküls nicht genutzt wird, und der hohe Salzanfall.

Aus Chemical Letters 575 bis 578 (1992) ist ein Verfahren zur N-Alkylierung von Pyrazolen bekannt, bei dem N-unsubstituierte Pyrazole mit Alkoholen in Gegenwart katalytischer Mengen von Ruthenium-, Rhodium- oder Iridium-Trialkylphosphit-Komplexen umgesetzt werden.

Nachteilig bei diesem Verfahren ist der hohe Preis für die Katalysatoren.

Aus DE-A-43 18 960 und DE-A-44 03 815 ist ein Verfahren zur N-Alkylierung von N-unsubstituierten Pyrazolen bekannt, bei dem N-unsubstituierte Pyrazole mit Alkoholen bzw. Ethern in Gegenwart von Heterogenkatalysatoren in der Gasphase umgesetzt werden.

Nachteilig bei diesem Verfahren ist, insbesondere bei sehr hochsiedenden N-unsubstituierten Pyrazolen, die Notwendigkeit, die Ausgangsstoffe zu verdampfen.

Aufgabe der vorliegenden Erfindung war daher, ein einfacheres und kostengünstigeres Verfahren zur Herstellung von N-substituierten Pyrazolen zu entwickeln.

Demgemäß wurde ein neues und verbessertes Verfahren zur Herstellung von N-substituierten Pyrazolen der allgemeinen Formel I 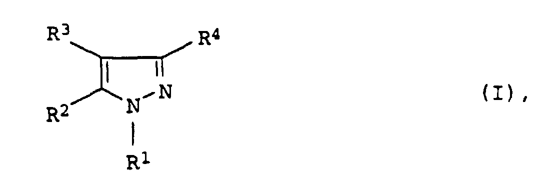 in der
- R¹: C₁- bis C₁₂-Alkyl oder C₇- bis C₂₀-Phenylalkyl und
- R², R³, R⁴: unabhängig voneinander Wasserstoff, C₁- bis C₁₂-Alkyl, C₇- bis C₂₀-Phenylalkyl oder gegebenenfalls substituierte Arylreste
bedeuten, durch Umsetzung von Pyrazolen der allgemeinen Formel II 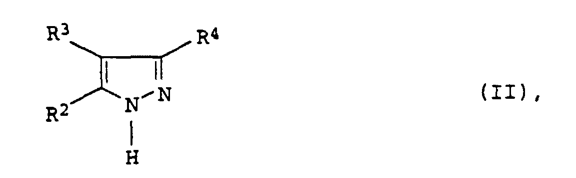 in der R²,R³ und R⁴ die obengenannten Bedeutungen haben, mit einem Alkohol der allgemeinen Formel III

R¹-O-H (III),

in der R¹ die obengenannten Bedeutungen hat, bei Temperaturen von 50 bis 400°C in Gegenwart eines Katalysators gefunden, welches dadurch gekennzeichnet ist, daß man die Umsetzung von Pyrazol II mit Verbindung III im Molverhältnis von 0,001:1 bis 1:1 in flüssiger Phase bei einem Unterdruck von 0,8 bar bis zu einem Überdruck von 250 bar durchführt und als Katalysator Säuren und/oder deren Alkylester bzw. deren Anhydride im Molverhältnis von 0,0001:1 bis 0,5:1 zum Pyrazol II einsetzt.

Die im Vergleich zu den N-unsubstituierten Pyrazolen II niedrigersiedenden N-substituierten Pyrazole I werden mit überschüssigem Alkylierungsreagenz III kontinuierlich gasförmig aus dem Reaktor ausgetragen, jedoch ist das Austragen des Reaktionsprodukts während der Reaktion nicht zwingend notwendig.

Das erfindungsgemäße Verfahren läßt sich wie folgt durchführen:

Die Umsetzung kann durch in Kontaktbringen von einem Pyrazol II und einem Alkohol III in Gegenwart eines beschriebenen Katalysators bei Temperaturen von 50 bis 400°C, bevorzugt 100 bis 350°C und einem Druck von 0,8 bis 250 bar, bevorzugt 0,8 bis 100 bar, besonders bevorzugt 0,9 bis 100 bar durchgeführt werden.

Das Molverhältnis von Pyrazol II zur Verbindung III beträgt in der Regel von 0,001:1 bis 1:1, bevorzugt 0,002:1 bis 1:1, besonders bevorzugt 0,003:1 bis 1:1.

Besonders bevorzugt wird die Umsetzung bei Temperaturen, einem Druck und Molverhältnissen durchgeführt, bei denen das Pyrazol II und der Katalysator in flüssiger Phase vorliegen und das N-substituierte Pyrazol I zusammen mit nicht umgesetzter Verbindung III gasförmig aus dem Reaktionsgefäß ausgetragen werden.

Das Pyrazol II und der Katalysator können in einem inerten Lösungsmittel, wie z.B. technischem Weißöl oder Vakuumgasöl, gelöst oder suspendiert vorgelegt werden.

Als Katalysatoren eignen sich Verbindungen, wie z.B. Schwefelsäure, Phosphorsäure, Alkyl- bzw. Arylsulfonsäuren bzw. deren Alkylester oder deren Anhydride. Das Molverhältnis von Katalysator zum Pyrazol II beträgt in der Regel von 0,0001:1 bis 0,5:1, bevorzugt 0,0005:1 bis 0,5:1, besonders bevorzugt 0,01:1 bis 0,2:1.

Im Vergleich zu den bekannten Verfahren liefert das erfindungsgemäße Verfahren auf einfacherem und wirtschaftlicherem Wege N-substituierte Pyrazole.

Als Ausgangsverbindung II eignen sich Pyrazole, wie Pyrazol und substituierte Pyrazole, 3-Methylpyrazol, 4-Methylpyrazol, 3,4-Dimethylpyrazol, 3,5-Dimethylpyrazol, 3,4,5-Trimethylpyrazol, 3-Ethylpyrazol, 4-Ethylpyrazol, 3-Arylpyrazole, 3-Phenylpyrazol, 3,5-Diarylpyrazole, 3,5-Diphenylpyrazol, 3,4-Diarylpyrazole, 3,4-Diphenylpyrazol, 3,4,5-Triarylpyrazole und 3,4,5-Triphenylpyrazol.

Als Ausgangsverbindung III eignen sich Alkohole, wie Methanol, Ethanol, n-Propanol, Isopropanol, n-Butanol, tert.-Butanol und n-Octanol etc.

Die nach dem erfindungsgemäßen Verfahren herstellbaren N-substituierten Pyrazole I sind wertvolle Ausgangsstoffe bei der Herstellung von Farbstoffen, Pharmazeutika und Pflanzenschutzmitteln.

Die Substituenten R¹, R², R³, R⁴ und R⁵ in den Verbindungen I, II und III haben folgende Bedeutungen:
- R¹,R²,R³,R⁴: unabhängig voneinander
- C₁- bis C₁₂-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁bis C₄-Alkyl wie Methyl, Ethyl, n-Propyl, iso-Propyl, n-Butyl, iso-Butyl, sec.-Butyl und tert.-Butyl,
- C₇- bis C₂₀-Phenylalkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- R², R³, R⁴: unabhängig voneinander
- Wasserstoff
- Aryl, bevorzugt Phenyl,
- substituierte Arylreste wie C₇- bis C₂₀-Alkylphenyl, bevorzugt C₇- bis C₁₂-Alkylphenyl wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl.

### Beispiele

### Beispiel 1

In einer Blasensäule wurden 50 g (0,2273 mol) 3,5-Diphenylpyrazol und 5 g (0,0437 mol) Phosphorsäure (85%ig) bei 250°C vorgelegt, innerhalb von 4 Stunden wurden insgesamt 158,2 g (4,94 mol) Methanol durchgeleitet und der gasförmige Austrag kondensiert. Anschließend wurden noch zweimal je 50 g (0,2273 mol) 3,5-Diphenylpyrazol nachgefüllt und wie zuvor mit jeweils 158,2 g (4,94 mol) Methanol umgesetzt und kondensiert. Anschließend wurden 31,5 g (0,143 mol) 3,5-Diphenylpyrazol nachgefüllt, erneut 158,2 g (4,94 mol) Methanol durchgeleitet und kondensiert. Danach wurden ohne 3,5-Diphenylpyrazol nachzufüllen 158,2 g (4,94 mol) Methanol durchgeleitet. Jeder dieser fünf Ansätze wurde getrennt gesammelt, gaschromatographisch analysiert und i.Vak. eingeengt.

| Ansatz Nr.: | Molverhältnis: 1-Methyl-3,5-diphenylpyrazol/3,5-Diphenylpyrazol | Austrag [g] |
|---|---|---|
| 1 | 94 : 6 | 29,4 |
| 2 | 96,9 : 3,1 | 46,4 |
| 3 | 97,8 : 2,2 | 47,1 |
| 4 | 94,7 : 5,3 | 35,3 |
| 5 | 100 : 0 | 23,7 |

Insgesamt wurden 181,5 g (0,825 mol) 3,5-Diphenylpyrazol und 791 g (24,7 mol) Methanol eingesetzt. Man erhielt insgesamt 175,8 g (91 %) 1-Methyl- 3,5-diphenylpyrazol. Der hold-up der Blasensäule wurde nicht berücksichtigt.

### Beispiel 2

In einer elektrisch beheizten Kolonne mit 4 cm Durchmesser - ausgestattet mit einer Fritte am unteren Ende, aufgesteckt auf einen 250 ml Zweihalskolben -, wurden innerhalb von 6 Stunden tropfenweise 308,5 g (9,64 Mol) Methanol bei 155°C verdampft und durch die 200°C heiße Schmelze aus 10 g (0,0455 Mol) 3,5-Diphenylpyrazol und 0,046 g (0,000455 Mol) konz. H₂SO₄ geleitet. Der gasförmige Reaktionsaustrag wurde kondensiert und i.Vak. eingeengt. Man erhielt 10,2 g (92,5%) 1-Methyl-3,5-diphenylpyrazol,
Kp.: 3 mbar/190°C, mit einem Gehalt von 96,6% (GC).

### Beispiel 3

Analog Beispiel 2 wurde eine Schmelze von 10 g (0,0455 Mol) 3,5-Diphenylpyrazol und 0,051 g (0,000455 Mol) Methylsulfat vorgelegt und innerhalb von 3 Stunden mit 197,75 g (6,18 Mol) gasförmigen (bei 160°C verdampften) Methanols umgesetzt. Man erhielt 9,7 g (86,7%) 1-Methyl-3,5-diphenylpyrazol,
Kp.: 3 mbar/190°C, mit einem Gehalt von 95,2% (GC).

### Beispiel 4

Analog Beispiel 2 wurde bei 200°C eine Schmelze bestehend aus 10 g (0,0455 Mol) 3,5-Diphenylpyrazol und 0,057 g (0,00455 Mol) Dimethylsulfat hergestellt. Innerhalb von 5 Stunden wurden 435 g (13,6 Mol) Methanol bei 155°C verdampft und durch die auf 200°C gehaltene Schmelze geleitet. Man erhielt 10,4 g (96,9%) 1-Methyl-3,5-diphenylpyrazol, Kp: 3 mbar/190°C, mit einem Gehalt von 99,2% (GC).

### Beispiel 5

10 g (0,122 Mol) 4-Methylpyrazol, 1,25 g (0,0122 Mol) Schwefelsäure und 20 g Glas-Raschigringen mit 3 mm Durchmesser wurden analog Beispiel 2 auf 145°C erhitzt und innerhalb von 5 Stunden mit 118,65 g (3,7 Mol) bei 170°C verdampften Methanols bei 145°C umgesetzt. Nach Einengung bei Normaldruck erhielt man 11,2 g (94,4%) 1,4-Dimethylpyrazol, Kp.: 151°C, mit einem Gehalt von 98, 6 % (GC).

### Beispiel 6

Analog Beispiel 5 wurden 10 g (0,122 Mol) 4-Methylpyrazol mit 1,37 g (0,0122 Mol) Methylsulfat auf 190°C erwärmt und innerhalb von 3 Stunden mit 79,1 g (2,47 Mol) bei 158°C verdampften Methanols umgesetzt. Man erhielt 11,5 g (93,5%) 1,4-Dimethylpyrazol, Kp.: 151°C, mit einem Gehalt von 95,2% (GC).

### Beispiel 7

Analog Beispiel 5 wurden 10 g (0,122 Mol) 4-Methylpyrazol mit 1,54 g (0,0122 Mol) Dimethylsulfat auf 195°C erwärmt und innerhalb von 3 Stunden mit 118,72 g (3,71 Mol) bei 155°C verdampften Methanols umgesetzt. Man erhielt 11,6 g (95,4%) 1,4-Dimethylpyrazol, Kp.: 151°C, mit einem Gehalt von 96,3% (GC).

### Beispiel 8

Analog Beispiel 5 wurden 10 g (0,122 Mol) 4-Methylpyrazol mit 2 g (0,025 Mol) Schwefeltrioxid auf 160°C erwärmt und innerhalb von 4 Stunden mit 180 g (5,63 Mol) bei 130°C verdampften Methanols umgesetzt. Man erhielt 10,6 g (84,9%) 1,4-Dimethylpyrazol, Kp.: 151°C, mit einem Gehalt von 93,8% (GC).

### Beispiel 9

Analog Beispiel 5 wurden 10 g (0,122 Mol) 4-Methylpyrazol mit 2 g (0,014 Mol) Phosphorpentoxid auf 150°C erwärmt und innerhalb von 3,5 Stunden mit 175 g (5,5 Mol) bei 125°C verdampften Methanols umgesetzt. Man erhielt 10,9 g (89,5%) 1,4-Dimethylpyrazol, Kp.: 151°C, mit einem Gehalt von 96,2% (GC).

### Beispiel 10

Analog Beispiel 2 wurde eine Schmelze von 10 g (0,0455 Mol) 3,5-Diphenylpyrazol und 0,72 g (0,00455 Mol) Benzolsulfonsäure vorgelegt und innerhalb von 4,5 Stunden mit 245 g (7,66 Mol) gasförmigen (bei 160°C verdampften) Methanols umgesetzt. Man erhielt 9,6 g (88,5%) 1-Methyl-3,5-diphenylpyrazol, Kp.: 3 mbar/190°C, mit einem Gehalt von 98,2% (GC).

### Beispiel 11

Analog Beispiel 2 wurde eine Schmelze von 10 g (0,0455 Mol) 3,5-Diphenylpyrazol und 0,44 g (0,0045 Mol) Methansulfonsäure vorgelegt und innerhalb von 4 Stunden mit 175 g (5,46 Mol) gasförmigen (bei 160°C verdampften) Methanols umgesetzt. Man erhielt 10 g (92%) 1-Methyl-3,5-diphenylpyrazol, Kp: 3 mbar/190°C, mit einem Gehalt von 98% (GC).

### Beispiel 12

Analog Beispiel 2 wurde die Kolonne mit 30 g Glasringen vom Durchmesser 3 mm, 16,4 g (0,24 Mol) Pyrazol und 1,02 g (0,01 Mol) 96 Gew.-%iger Schwefelsäure gefüllt, auf 195°C erhitzt und innerhalb von 7 Stunden mit 703 g (9,5 Mol) bei 180°C verdampftem tert.-Butanol umgesetzt. Man erhielt 21,7 g N-tert.-Butylpyrazol, Kp: 103°C, mit einem Gehalt von 99,4% (GC), was einer Ausbeute von 72,5% entspricht.

### Beispiel 13

20,5 g (0,25 Mol) 4-Methylpyrazol, 82 g (0,65 Mol) n-Octanol und 1,28 g (0,0125 Mol) 96 Gew.-%iger Schwefelsäure wurden 33 Stunden bei 175°C gerührt. Das Reaktionsgemisch wurde nach dem Abkühlen dreimal mit jeweils 15 ml 5%-iger Schwefelsäure extrahiert.

Die vereinigten Schwefelsäureextrakte wurden mit 25%-iger Natronlauge neutralisiert. Die anschließende Destillation lieferte 13,5 g, nach GC-Analyse 99,3%-iges nicht umgesetztes 4-Methylpyrazol.

Aus der organische Phase erhielt man 17,4 g 4-Methyl-N-octylpyrazol, Kp.: 75°C/7 mbar, mit einem Gehalt von 95% (GC) bei einem Umsatz von 34,6% und einer Selektivität von 98,5%.

### Beispiel 14

6,8 g (0,1 Mol) Pyrazol, 23,7 g (0,32 Mol) tert.-Butanol und 0,51 g (0,005 Mol) 96 Gew.-%iger Schwefelsäure wurden in einem Autoklaven 3 Stunden bei 200°C und 35 bar gerührt. Nach Abkühlen, Entspannen (Ausgasen von Isobutylen) und Phasentrennung erhielt man aus der wäßrigen Phase 6,5 g einer nach GC-Analyse 10,2 gew.-%igen wässrigen Pyrazollösung, deren Destillation 0,66 g Pyrazol lieferte. Aus der organischen Phase erhielt man 10,5 g (83,6%) N-tert.-Butylpyrazol, Kp.: 102°C, mit einem Gehalt von 98,8% (GC).

### Beispiel 15

34 g (0,5 Mol) Pyrazol und 83 g (0,64 Mol) 2-Ethyl-1-hexanol wurden mit 2,55 g (0,025 Mol) 96 Gew.-%iger Schwefelsäure 20 Stunden bei 175°C gerührt. Das Reaktionsgemisch wurde nach dem Abkühlen dreimal mit jeweils 15 ml 5%-iger Schwefelsäure extrahiert. Die vereinigten Schwefelsäureextrakte wurden mit 25%-iger Natronlauge neutralisiert. Die anschließende Destillation lieferte 27,0 g, nach GC-Analyse 99,1%-iges nicht umgesetztes Pyrazol. Aus der organische Phase erhielt man 19,6 g N-Hexyl-2-ethylpyrazol, Kp.: 65°C/50 mbar, mit einem Gehalt von 92% (GC) bei einem Umsatz von 21,3% und einer Selektivität von 93,9%.

## Patentansprüche

1. Verfahren zur Herstellung von N-substituierten Pyrazolen der allgemeinen Formel I 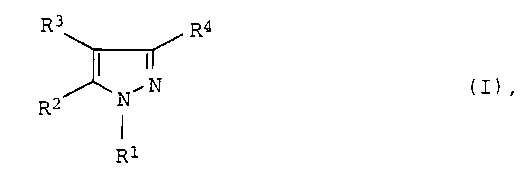 in der
R¹ C₁- bis C₁₂-Alkyl oder C₇- bis C₂₀-Phenylalkyl und
R², R³, R⁴ unabhängig voneinander Wasserstoff, C₁- bis C₁₂-Alkyl, C₇- bis C₂₀-Phenylalkyl oder gegebenenfalls substituierte Arylreste
bedeuten, durch Umsetzung von Pyrazolen der allgemeinen Formel II 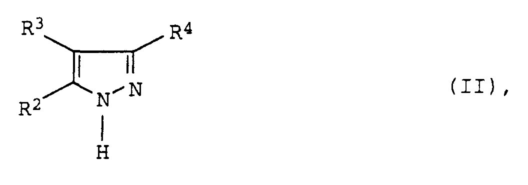 in der R²,R³ und R⁴ die obengenannten Bedeutungen haben, mit einem Alkohol der allgemeinen Formel III
R¹-O-H (III),
in der R¹ die obengenannten Bedeutungen hat, bei Temperaturen von 50 bis 400°C in Gegenwart eines Katalysators, **dadurch gekennzeichnet, daß** man die Umsetzung von Pyrazol II mit Verbindung III im Molverhältnis von 0,001:1 bis 1:1 in flüssiger Phase bei einem Unterdruck von 0,8 bar bis zu einem Überdruck von 250 bar durchführt und als Katalysator Säuren und/oder deren Alkylester bzw. deren Anhydride im Molverhältnis von 0,0001:1 bis 0,5:1 zum Pyrazol II einsetzt.

2. Verfahren zur Herstellung von N-substituierten Pyrazolen der Formel I nach Anspruch 1, **dadurch gekennzeichnet, daß** man als Katalysator Schwefelsäure, Alkylsulfate, Dialkylsulfate, Phosphorsäure, Phosphorsäurealkylester, Alkylsulfonsäuren oder deren Alkylester, Arylsulfonsäuren oder deren Alkylester sowie die Anhydride der genannten Säuren wie z.B. Schwefeltrioxid oder Phosphorpentoxid einsetzt.

3. Verfahren zur Herstellung von N-substituierten Pyrazolen der Formel I nach Anspruch 1, **dadurch gekennzeichnet, daß** man das Pyrazol II zur Verbindung III im Molverhältnis von 0,002:1 bis 1:1 einsetzt.

4. Verfahren zur Herstellung von N-substituierten Pyrazolen der Formel I nach Anspruch 1, **dadurch gekennzeichnet, daß** man das Pyrazol II zur Verbindung III im Molverhältnis von 0,003:1 bis 1:1 einsetzt.

5. Verfahren zur Herstellung von N-substituierten Pyrazolen der Formel I nach Anspruch 1, **dadurch gekennzeichnet, daß** man den Katalysator zum Pyrazol II im Molverhältnis von 0,0005:1 bis 0,5:1 einsetzt.

6. Verfahren zur Herstellung von N-substituierten Pyrazolen der Formel I nach Anspruch 1, **dadurch gekennzeichnet, daß** man den Katalysator zum Pyrazol II im Molverhältnis von 0,01:1 bis 0,2:1 einsetzt.

7. Verfahren zur Herstellung von N-substituierten Pyrazolen der Formel I nach Anspruch 1, **dadurch gekennzeichnet, daß** man die Umsetzung bei einem Druck von 0,8 bis 100 bar durchführt.

8. Verfahren zur Herstellung von N-substituierten Pyrazolen der Formel I nach Anspruch 1, **dadurch gekennzeichnet, daß** man die Umsetzung bei einem Druck von 0,9 bis 100 bar durchführt.

9. Verfahren zur Herstellung von N-substituierten Pyrazolen der Formel I nach Anspruch 1, **dadurch gekennzeichnet, daß** man die Umsetzung bei Temperaturen von 100 bis 350°C durchführt.

## Claims

1. A process for the preparation of N-substituted pyrazoles of the general formula I 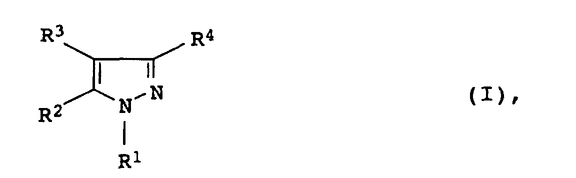 where
R¹ is C₁-C₁₂-alkyl or C₇-C₂₀-phenylalkyl and
R², R³ and R⁴ are, independently of one another, hydrogen, C₁-C₁₂-alkyl, C₇-C₂₀-phenylalkyl or unsubstituted or substituted aryl radicals,
by reacting pyrazoles of the general formula II 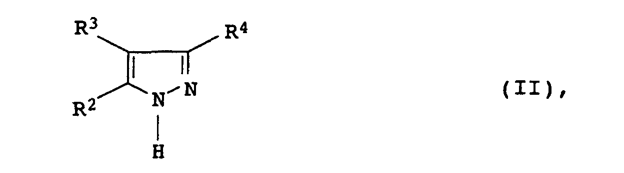 where R², R³ and R⁴ have the abovementioned meanings, with an alcohol of the general formula III
R¹-O-H (III),
where R¹ has the abovementioned meanings, at from 50 to 400°C in the presence of a catalyst, wherein the reaction of pyrazole II with compound III in the molar ratio of from 0.001:1 to 1:1 is carried out in liquid phase under a subatmospheric pressure of 0.8 bar up to a superatmospheric pressure of 250 bar, and acids and/or their alkyl esters or their anhydrides are used, in the molar ratio of from 0.0001:1 to 0.5:1 with respect to the pyrazole II, as catalyst.

2. A process for the preparation of N-substituted pyrazoles of the formula I as claimed in claim 1, wherein sulfuric acid, alkyl sulfates, dialkyl sulfates, phosphoric acid, alkyl phosphates, alkylsulfonic acids or their alkyl esters, arylsulfonic acids or their alkyl esters, and the anhydrides of said acids such as sulfur trioxide or phosphorus pentoxide is used as catalyst.

3. A process for the preparation of N-substituted pyrazoles of the formula I as claimed in claim 1, wherein the molar ratio of pyrazole II to compound III is from 0.002:1 to 1:1.

4. A process for the preparation of N-substituted pyrazoles of the formula I as claimed in claim 1, wherein the molar ratio of pyrazole II to compound III is from 0.003:1 to 1:1.

5. A process for the preparation of N-substituted pyrazoles of the formula I as claimed in claim 1, wherein the molar ratio of catalyst to the pyrazole II is from 0.0005:1 to 0.5:1.

6. A process for the preparation of N-substituted pyrazoles of the formula I as claimed in claim 1, wherein the molar ratio of catalyst to the pyrazole II is from 0.01:1 to 0.2:1.

7. A process for the preparation of N-substituted pyrazoles of the formula I as claimed in claim 1, wherein the reaction is carried out under a pressure of from 0.8 to 100 bar.

8. A process for the preparation of N-substituted pyrazoles of the formula I as claimed in claim 1, wherein the reaction is carried out under a pressure of from 0.9 to 100 bar.

9. A process for the preparation of N-substituted pyrazoles of the formula I as claimed in claim 1, wherein the reaction is carried out at from 100 to 350°C.

## Revendications

1. Procédé de préparation de pyrazoles N-substitués de la formule générale I 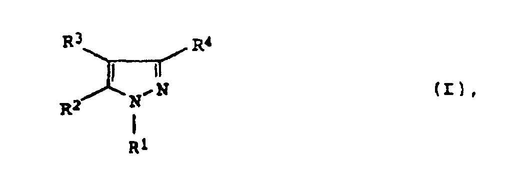 dans lequel
R¹ signifie alkyle en C₁ à C₁₂ ou phénylalkyle en C₇ à C₂₀
R², R³, R⁴ signifient indépendamment l'un de l'autre de l'hydrogène, de l'alkyle en C₁ à C₁₂, du phénylalkyle en C₇ à C₂₀ ou le cas échéant, des radicaux substitués d'aryle
par transformation de pyrazoles de la formule générale II 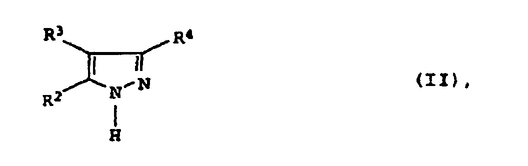 dans laquelle R², R³ et R⁴ ont la signification susmentionnée, avec un alcool de la formule générale III
R¹-O-H (III),
dans laquelle R¹ a la signification susmentionnée, à des températures comprises entre 50 et 400 °C, en présence d'un catalyseur, **caractérisé en ce que** la transformation de pyrazoles II avec un composé III est réalisée selon un rapport molaire compris entre 0,001 : 1 et 1 :1, en phase liquide, sous une dépression de 0,8 bar, jusqu'à une surpression de 250 bar et **en ce qu'**on utilise en tant que catalyseur des acides et/ou les esters alkyles de ces derniers ou leurs anhydrides, selon un rapport molaire compris entre 0,0001 : 1 et 0,5 : 1 par rapport au pyrazole II.

2. Procédé de préparation de pyrazoles N-substitués, de la formule I selon la revendication 1, **caractérisé en ce que** le catalyseur utilisé est de l'acide sulfurique, du sulfate d'alkyle, du sulfate de dialkyle, de l'acide phosphorique, de l'ester d'alkyle d'acide phosphorique, des acides d'alkyle sulfoniques ou les esters d'alkyle de ces derniers, des acides d'aryles sulfoniques ou les esters d'aryle de ces derniers, ainsi que les anhydrides des acides cités, comme par exemple le trioxyde de soufre ou le pentoxyde de phosphore.

3. Procédé de préparation de pyrazoles N-substitués de la formule I selon la revendication 1, **caractérisé en ce que** le pyrazole II est utilisé, selon un rapport molaire compris entre 0,002 : 1 et 1 : 1 par rapport au composé III.

4. Procédé de préparation de pyrazoles N-substitués de la formule I selon la revendication 1, **caractérisé en ce que** le pyrazole II est utilisé, selon un rapport molaire compris entre 0,003 : 1 et 1 : 1 par rapport au composé III.

5. Procédé de préparation de pyrazoles N-substitués de la formule I selon la revendication 1, **caractérisé en ce que** le catalyseur est utilisé selon un rapport molaire compris entre 0,0005 : 1 et 0,5 : 1 par rapport au pyrazole II.

6. Procédé de préparation de pyrazoles N-substitués de la formule I selon la revendication 1, **caractérisé en ce que** le catalyseur est utilisé, selon un rapport molaire compris entre 0,01 : 1 et 0,2 : 1 par rapport au pyrazole II.

7. Procédé de préparation de pyrazoles N-substitués de la formule I selon la revendication 1, **caractérisé en ce que** la transformation est réalisée sous une pression comprise en 0,8 et 100 bar.

8. Procédé de préparation de pyrazoles N-substitués de la formule I selon la revendication 1, **caractérisé en ce que** la transformation est réalisée sous une pression comprise entre 0,9 et 100 bar.

9. Procédé de préparation de pyrazoles N-substitués de la formule I selon la revendication 1, **caractérisé en ce que** la transformation est réalisée à des températures comprises entre 100 et 350 ° C.
